(19) Europäisches Patentamt
European Patent Office
Office européen des brevets

(11) Veröffentlichungsnummer: **0 222 117**
**B1**

(12) # EUROPÄISCHE PATENTSCHRIFT

(45) Veröffentlichungstag der Patentschrift: **10.01.90**

(51) Int. Cl.⁴: **C07C 29/14**, C07C 33/14, C07D 309/24, B01J 31/14

(21) Anmeldenummer: **86113117.5**

(22) Anmeldetag: **24.09.86**

(54) **Verfahren zur Herstellung von ungesättigten cyclischen Alkoholen.**

(30) Priorität: **11.11.85 DE 3539872**

(43) Veröffentlichungstag der Anmeldung:
**20.05.87 Patentblatt 87/21**

(45) Bekanntmachung des Hinweises auf die Patenterteilung:
**10.01.90 Patentblatt 90/2**

(84) Benannte Vertragsstaaten:
**AT CH DE FR GB IT LI NL SE**

(56) Entgegenhaltungen:
**EP-A- 0 097 940**

**JOURNAL OF ORGANIC CHEMISTRY, Band 23, 1958, Seiten 184-186, rechte Spalte, Zeilen 21-35, Baltimore, US; R. ZELINSKI et al.: "Racemic 2-Hydroxymethyl-2,3-dihydro-4H-pyran, a model carbohydrate"**
**THE JOURNAL OF THE AMERICAN CHEMICAL SOCIETY, Band 64, 1942, Seiten 1497-1499, Columbus, Ohio, US; H.E. FRENCH et al.: "The action of alkali on cyclohexene carbonals"**

(73) Patentinhaber: **Degussa Aktiengesellschaft, Weissfrauenstrasse 9, D-6000 Frankfurt am Main 1(DE)**

(72) Erfinder: **Grund, Andreas, Dr. Dipl.-Chem., Rilkeweg 15, D-6100 Darmstadt(DE)**
Erfinder: **Prescher, Günther, Dr. Dipl.-Chem., Liesingstrasse 2, D-6450 Hanau 9(DE)**
Erfinder: **Ecker, Armin, Südring 49, D-6458 Rodenbach(DE)**

**EP 0 222 117 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft ein Verfahren zur Herstellung ungesättigter cyclischer Alkohole durch Reduktion der entsprechenden Aldehyde in Gegenwart von Aluminiumalkoholat und 2-Propanol. Es sind verschiedene Methoden bekannt, ungesättigte cyclische Alkohole durch Reduktion zu gewinnen.

So betrifft die DE-PS 1 101 409 die Hydrierung ungesättigter Aldehyde in Gegenwart von Kupferchromit. Wegen der hohen notwendigen Drücke von 100 bis 200 bar ist eine erhebliche apparative Ausrüstung notwendig, welche gerade bei kontinuierlicher Fahrweise erhebliche Kosten mit sich bringt.

Weiterhin ist bekannt, obenstehende Aldehyde mit komplexen Hydriden, z.B. Natriumborhydrid oder Lithium-Aluminiumhydrid zu reduzieren (US-PS 4 287 100 und J.Org.Chem. 23, 184 (1958)).

Neben den kostspieligen Reduktionsmitteln verhindern die verwendeten Lösungsmitteln wie Ether, Tetrahydrofuran wegen ihrer leichten Entzündbarkeit eine technische Verwertung dieses Verfahrens.

Eine weitere Alternative besteht darin, den ungesättigten Aldehyd im Sinne einer Tischtschenko-Reaktion zu verestern und den Ester anschließend hydrolytisch zu spalten (C.W.Smith, Acrolein, Hüthig, Heidelberg, 1975, S. 176).

Dieses Verfahren ist besonders unwirtschaftlich, da zum einen ein zusätzlicher Reaktionsschritt vorgelagert ist, und zum anderen, weil die Hälfte des eingesetzten Aldehyds als Carbonsäure bzw. deren Salz anfällt.

Es ist auch bekannt Aldehyde nach Meerwein-Ponndorf Verley mit Aluminiumisopropylat zu reduzieren. Man setzt dabei einen großen Überschuß an Isopropylat ein, wie man den folgenden Handbüchern entnehmen kann:

Vogel's Textbook of Practical Organic Chemistry, Longman, London, New York, 1978, 4. Auflage, S. 454;

Organic Reactions, Volume II, John Wiley & Sons, 4. Auflage 1947, S. 195.

Aus der EP-A 0 097 040 ist ein Verfahren zur Herstellung araliphatischer Alkohole bekannt, bei dem die entsprechenden Aldehyde oder Kentone nach Meerwein-Ponndorf-Verley mit Aluminiumalkoholaten reduziert werden, deren Menge sich vorzugsweise auf etwa 0,01 bis 0,33 Mol pro Mol Aldehyd oder Keton beläuft. Das Verfahren wird gegebenenfalls in Gegenwart eines Alkohols, vorzugsweise des dem Aluminiumalkoholat zugrundeliegenden Alkohols, durchgeführt.

Die Reduktion von Δ3-Tethrahydrobenzaldehyd erfolgt dagegen unter Anwendung eines Überschusses an Isopropylat (Ber. Dtsch. Chem. Ges. 75, (1942) 890, 891).

Gleichzeitig ist darauf zu verweisen, daß ebenfalls Lösungsmittel (2-Propanol) in großen Mengen, bezogen auf den Aldehyd, verwendet wird.

Aufgabe der Erfindung ist ein Verfahren zur Herstellung von ungesättigten cyclischen Alkoholen auf einem wirtschaftlichen Weg durch Reduktion der entsprechenden Aldehyde.

Gegenstand der Erfindung ist ein Verfahren zur Herstellung von ungesättigten cyclischen Alkoholen der Formel (I)

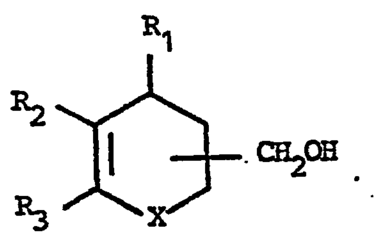

,

in der bedeuten

$R_1$, $R_2$, $R_3$, gleich oder verschieden: Wasserstoff, ein $C_1$–$C_{10}$ Alkylrest, geradkettig oder verzweigt, $C_2$–$C_6$ Alkenyl, geradkettig oder verzweigt, X: Sauerstoff oder $>CHR_4$, wobei $R_4$ die gleiche Bedeutung wie $R_1$, $R_2$, $R_3$ hat, oder $-CH<$, wenn über $R_1 = -CH_2-$ eine Kohlenstoffbrücke zu X besteht,

durch Reduktion der entsprechenden Aldehyde unter Verwendung von Aluminiumalkoholaten und 2-Propanol, dadurch gekennzeichnet, daß man den Aldehyd bei erhöhter Temperatur mit der 1,5 bis 5-fachen, bevorzugt 1,5 bis 3-fachen molaren Menge 2-Propanol in Gegenwart von 0,1 bis 5 Gew.-% Aluminiumalkoholat, beides bezogen auf den Aldehyd, reduziert, das bei der Reduktion entstehende Aceton gleichzeitig abdestilliert und anschließend den ungesättigten Alkohol destillativ reinigt.

Geringere Mengen 2-Propanol führen zu schlechten Ausbeuten, größere zu einem erhöhten Destillationsaufwand.

Eingesetzt werden Aldehyde der Formel (II)

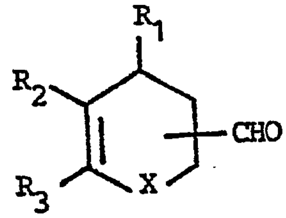

in der $R_1$, $R_2$, $R_3$ und X die oben angegebene Bedeutung haben.
Die Carboxaldehydgruppe befindet sich entweder in α- oder in β-Stellung, bevorzugt in α-Stellung, bezogen auf X.
Der Ring ist zusätzlich zur Carboxaldehydgruppe bevorzugt nur einfach substituiert, vorteilhaft aber nicht dieser Gruppe benachbart.
Eine Ausnahme stellt die Brückenbildung zwischen $R_1$ und X dar.

Besonders sind als Substituenten die Methylgruppe und 4-Methyl-3-pentenylgruppe geeignet.

Als vorteilhaft erweist sich insbesondere die Verwendung von
Norbonen-(2)-aldehyd,
2,3-Dihydro-4H-pyran-2-carboxaldehyd
(Formyldihydropyran)
3-Cyclohexen-1-carboxaldehyd
3- bzw. 4-(4-Methyl-3-pentenyl)-3-cyclohexen
-1-carboxaldehyd(Myracaldehyd)
3-bzw. 4-Methyl-3-cyclohexen-1-carboxaldehyd.

Man führt die Reaktion diskontinuierlich beispielsweise in einer trockenen Destillationsapparatur durch, in der man das Reaktionsgemisch erhitzt und das entstandene Aceton gegebenenfalls zusammen mit geringen Mengen 2-Propanol laufend abzieht.

Dabei liegt die Umsetzungstemperatur zwischen 80 und 120°C unter Normaldruck.

Dabei ist natürlich zu berücksichtigen, daß bei erhöhten Drücken höhere Temperaturen und bei Drücken unterhalb des Normaldrucks tiefere Temperaturen vorteilhaft sind.

Eine Variante besteht darin, daß man das Gemisch nicht vorlegt, sondern den Aldehyd entweder allein oder in 2-Propanol gelöst in die siedende Lösung des Alkoholats in 2-Propanol eindosiert.

Nach Abschluß der Reaktion zieht man das restliche 2-Propanol unter Vakuum ab und gewinnt anschließend destillativ oder durch Extraktion den gewünschten ungesättigten Alkohol.

Seine besonderen Vorteile entfaltet das erfindungsgemäße Verfahren bei kontinuierlicher Durchführung.

Das überschüssige 2-Propanol kann zum Beispiel, da kein Wasser und keine Säure zur Zerstörung des Alkoholats eingesetzt werden muß, in einfacher Weise abgezogen und der Reduktion wieder zugeführt werden.

Man geht so vor, daß man oberhalb des Sumpfes 2-Propanol, Aluminiumalkoholat und Aldehyd in die Kolonne einspeist und über Kopf das als Nebenprodukt anfallende Aceton, das ggf. Spuren an 2-Propanol enthalten kann, abzieht. Der Sumpf, der vorwiegend aus 2-Propanol, Alkoholat und dem ungesättigten Alkohol neben geringen Mengen Aldehyd und Hochsiedern besteht, wird in eine zweite Kolonne eingeleitet, in der man als Kopfprodukt 2-Propanol entfernt. Im Sumpf der zweiten Kolonne fällt dann als Rohprodukt ein Gemisch aus gewünschtem Alkohol, Hochsiedern und dem Aluminiumalkoholat an.

Aus diesem Gemisch trennt man in einer dritten Kolonne den ungesättigten Alkohol ab.

Das in der 2. Kolonne über Kopf anfallende 2-Propanol wird nach Ergänzung gemäß der gebildeten Acetonmenge in die Reduktionskolonne zurückgeführt. Besonders vorteilhaft kann die Aufarbeitung dadurch gestaltet werden, indem man in Kolonne 2 den ungesättigten Alkohol dampfförmig im Seitenstrom über dem Sumpf oder oberhalb der ersten Böden entnimmt und damit die 3. Kolonne einspart.

Kolonne 2 und Kolonne 3 werden bei Normaldruck betrieben; ebenso gut kann aber auch unter Vakuum, zum Beispiel 100-500 mbar gearbeitet werden. Die Kolonnen können Bodenkolonnen sein, es können aber Kolonnen mit anderen Einbauten oder Füllkörperkolonnen, wie sie dem Fachmann bekannt sind, eingesetzt werden.

Die Einspeisung der Reaktionsteilnehmer erfolgt einzeln oder im Gemisch miteinander (außer Alkoholat und Aldehyd) und wird so gesteuert, daß ein möglichst hoher Umsatz erreicht wird.

Vorteilhafterweise führt man den Aldehyd im Gemisch mit 2-Propanol an der Einspeisungsstelle, gegebenenfalls auf 30 bis 70°C vorgeheizt zu.

Die Reduktion findet dann in der Kolonne innerhalb eines Temperaturbereichs statt, der bei Normaldruck von ca. 56°C am Kopf der Kolonne bis zu 80 bis 120°C im Sumpf reicht.

Die Sumpftemperaturen in der zweiten und dritten Kolonne werden durch die Siedetemperaturen des 2-Propanols beziehungsweise des gewünschten ungesättigten Alkohols bestimmt (in Abhängigkeit vom angewandten Druck).

Als Katalysator verwendet man dem Fachmann bekannte Aluminiumalkoholate, z.B. 2-Propanolat oder 2-Butanolat.

Feste Alkoholate löst man zweckmäßigerweise in 2-Propanol. Besonders bevorzugt ist der Einsatz eines bei Raumtemperatur flüssigen sog. Mischalkoholats des 2-Propanol und 2-Butanol, wie es zum Beispiel von Texaco kommerziell angeboten wird.

Es ist besonders günstig, einen Teil des Sumpfes der letzten Stufe, der vorwiegend Katalysator neben Hochsiedern enthält, in die Reduktionskolonne(erste Kolonne), zurückzuführen, um den Verbrauch an frischem Katalysator weiter zu verringern.

Die in diesem Sumpf enthaltenen Hochsieder bestehen vorwiegend aus den durch Tischtschenko-Reaktion gebildeten Estern sowie aus 2-Propyl und 2-Butyl-Estern der dem Aldehyd entsprechenden Carbonsäuren. Der Anteil an diesen Nebenprodukten ist nach dem erfindungsgemäßen Verfahren sehr gering, er liegt bei ca. 0,8 bis 3,0 Gew.-%, bezogen auf eingesetzten Aldehyd. Die nach dem erfindungsgemäßen Verfahren erhaltenen ungesättigten Alkohole fallen in hoher Reinheit an; sie sind wertvolle Vorstufen zur Herstellung von Epoxidharzen, Polymeren, Riechstoffen, pharmazeutischen Produkten und Pflanzenschutzmitteln.

<u>Beispiele:</u>

Allgemeine Versuchsbeschreibung:

In eine Glockenbodenkolonne (20 Böden) speist man Katalysator, Aldehyd und 2-Propanol ein (siehe Tabelle). Der Sumpf wurde kontinuierlich über einen Zwischenkühler abgezogen und in eine zweite Kolonne (Einlauf 10./11. Boden, p=300 mbar) eingespeist. Die Produktabnahme erfolgte dampfförmig im Seitenstrom zwischen dem 2./3. Boden. Das Destillat von Kolonne 2 (Kopfprodukt) wurde vollständig in die Beschickungsvorlage von Kolonne 1, der Sumpf zu bis 70 % in die Mischalkoholatvorlage rückgeführt.

Es wurden sowohl Gemische von Aldehyd und 2-Propanol (gleiche Bödenzahl) als auch die Komponenten einzeln zugeführt (ungleiche Bodenzahl).

Als Katalysator diente Aluminium-Mischalkoholat. Er wurde ebenfalls oberhalb des Sumpfes eingespeist.

Die Sumpftemperatur in der 1. Kolonne lag zwischen 85 und 110°C.
Das im Seitenstrom abgezogene Produkt bedurfte keiner weiteren Reinigung.
Es wurden eingesetzt:

A: 3-Cyclohexen-1-carboxaldehyd
B: Norbonen-2-aldehyd
C: Formyldihydropyran
D: Myracaldehyd

Die in der Tabelle angegebenen Verweilzeiten gelten für Kolonne 1 incl. Sumpf.
Umsatz und Ausbeute beziehen sich auf die Menge des eingesetzten Aldehyds.

**Tabelle**

| Beispiel Nr. | Aldehyd | Einspeisung mol/h Aldeh./ 2-Propanol | Gew.-% Katalysator | Verweilzeit [h] | Einspeisung auf Boden-nr. Aldeh. | 2-Propanol | Umsatz % | zu Hochsiedern, % | Ausbeute % |
|---|---|---|---|---|---|---|---|---|---|
| 1 | A | 0,930 : 1,892 | 2,0 | 2,94 | 10 | 10 | 99,57 | 2,58 | 82,47 |
| 2 | A | 0,941 : 1,908 | 2,0 | 2,91 | 10 | 10 | 99,68 | 3,19 | 83,74 |
| 3 | A | 0,768 : 1,717 | 2,0 | 5,23 | 15 | 16 | 98,44 | 2,08 | 95,4 |
| 4 | A | 0,930 : 1,828 | 1,0 | 4,65 | 15 | 16 | 98,92 | 2,15 | 82,47 |
| 5 | A | 0,930 : 1,815 | 1,0 | 4,66 | 15 | 16 | 87,85 | 3,01 | 76,02 |
| 6 | A | 0,923 : 3,90 | 1,0 | 2,94 | 15 | 16 | 98,27 | 1,54 | 88,52 |
| 7 | B | 0,993 : 1,869 | 2,0 | 4,23 | 15 | 16 | 90,53 | 3,22 | 67,07 |
| 8 | C | 1,047 : 1,874 | 2,0 | 4,29 | 15 | 16 | 91,31 | 0,76 | 75,55 |
| 9 | D | 0,85 : 1,93 | 1,6 | 3,82 | 15 | 16 | 98,47 | 2,28 | 85,9 |

## Patentansprüche

1. Verfahren zur Herstellung von ungesättigten cyclischen Alkoholen der Formel (I)

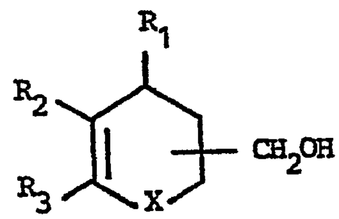

in der bedeuten
$R_1$, $R_2$, $R_3$, gleich oder verschieden: Wasserstoff, ein $C_1$–$C_{10}$ Alkylrest, geradkettig oder verzweigt, $C_2$–$C_6$-Alkenyl, geradkettig oder verzweigt, X: Sauerstoff oder >$CHR_4$, wobei $R_4$ die gleiche Bedeutung wie $R_1$, $R_2$, $R_3$ hat, oder –CH<, wenn über $R_1$ = –$CH_2$– eine Kohlenstoffbrücke zu X besteht,
durch Reduktion der entsprechenden Aldehyde unter Verwendung von Aluminiumalkoholaten und 2-Propanol, dadurch gekennzeichnet, daß man den Aldehyd bei erhöhter Temperatur mit der 1,5 bis 5-fachen molaren Menge 2-Propanol in Gegenwart von 0,1 bis 6 Gew.-% Aluminiumalkoholat, beides bezogen auf den Aldehyd, reduziert, das bei der Reduktion entstehende Aceton gleichzeitig abdestilliert und anschließend den ungesättigten Alkohol destillativ reinigt.

2. Verfahren nach Anspruch 1, dadurch gekennzeichnet, daß man gemischt oder getrennt Aldehyd, 2-Propanol und Aluminiumalkoholat in eine Kolonne einspeist, das bei der Reduktion entstehende Aceton über Kopf entfernt, das Sumpfprodukt in eine zweite Kolonne überführt, über Kopf 2-Propanol abzieht, das Sumpfprodukt in eine dritte Kolonne einspeist und als Kopfprodukt den ungesättigten Alkohol gewinnt.

3. Verfahren nach Anspruch 2, dadurch gekennzeichnet, daß man den ungesättigten Alkohol in der zweiten Kolonne dampfförmig im Seitenstrom entnimmt.

4. Verfahren nach den Ansprüchen 1 bis 3, dadurch gekennzeichnet, daß man als Aluminiumalkoholat ein bei Raumtemperatur flüssiges Aluminium-Mischalkoholat des 2-Propanol und 2-Butanol verwendet.

## Claims

1. Process for the preparation of unsaturated cyclic alcohols of the formula (I)

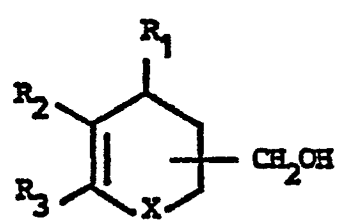

in which $R_1$, $R_2$ and $R_3$ are identical or different and denote: hydrogen, a straight-chain or branched $C_1$–$C_{10}$-alkyl radical or a straight-chain or branched $C_2$–$C_6$-alkenyl radical and X denotes: oxygen or >$CHR_4$, wherein $R_4$ has the same meaning as $R_1$, $R_2$ or $R_3$, or –CH<, if there is a carbon bridge to X via $R_1$ = –$CH_2$–,
by reduction of the corresponding aldehydes using aluminium alcoholates and 2-propanol, characterized in that the aldehyde is reduced at elevated temperature with 1.5 to 5 times the molar amount of 2-propanol in the presence of 0.1 to 6 wt.% aluminium alcoholate, both based on the aldehyde, the acetone formed during the reduction is simultaneously distilled off and the unsaturated alcohol is subsequently purified by distillation.

2. Process according to claim 1, characterized in that aldehyde, 2-propanol and aluminium alcoholate are fed into a column as a mixture or separately, the acetone formed during the reduction is removed via the top, the bottom product is transferred to a second column, 2-propanol is stripped off via the top, the bottom product is fed into a third column and the unsaturated alcohol is obtained as the top product.

3. Process according to claim 2, characterized in that the unsaturated alcohol in the second column is removed in vapour form in a side stream.

4. Process according to claims 1 to 3, characterized in that an aluminium mixed alcoholate of 2-propanol and 2-butanol which is liquid at room temperature is used as the aluminium alcoholate.

**Revendications**

1. Procédé d'obtention d'alcools cycliques non saturés de formule I,

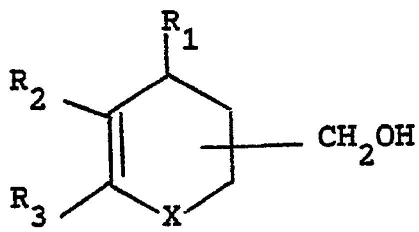

dans laquelle $R_1$, $R_2$ et $R_3$, indentiques ou différents, signifient de l'hydrogène, un radical alcoyle en $C_1$–$C_{10}$ en chaîne droite ou ramifiée, un radical alcényle en $C_2$–$C_6$ en chaîne droite ou ramifiée, X signifie de l'oxygène ou $>CH-R_4$ dans lequel $R_4$ a la même signification que $R_1$, $R_2$ et $R_3$ ou $-CH<$, lorsque par l'intermédiaire de $R_1 = -CH_2-$ il existe un pont carboné vers X, par réduction de l'aldéhyde correspondant en utilisant des alcoolates d'Aluminium et le 2-propanol, caractérisé en ce que l'on réduit l'aldéhyde à température plus élevée avec une quantité 1,5 à 5 fois molaire de 2-propanol, en présence de 0,1 à 6% en poids d'alcoolate d'Aluminium, les deux rapportées à l'aldéhyde, que l'on élimine par distillation, l'acétone formée au cours de la réaction en même temps et qu'ensuite on purifie par distillation l'alcool non saturé.

2. Procédé selon la revendication 1 caractérisé en ce que l'on introduit l'aldéhyde en mélange ou séparément, le 2-propanol et l'alcoolate d'Aluminium dans une colonne, que l'on élimine l'acétone qui survient au cours de la réduction par l'intermédiaire de la tête de la colonne, que l'on transfère le produit du bassin du pied dans une deuxième colonne, on soutire le 2-propanol par l'intermédiaire de la tête de colonne, on introduit le produit du bassin du pied dans une troisième colonne et on obtient l'alcool non saturé comme produit de tête.

3. Procédé selon la revendication 2 caractérisé en ce que l'on élimine l'alcool non saturé dans la deuxième colonne sous forme de vapeur dans le courant parallèle.

4. Procédé selon les revendications 1 à 3 caractérisé en ce que l'on utilise comme alcoolate d'Aluminium un alcoolate mixte liquide à température ordinaire à base de 2-propanol et de 2-butanol.